# EUROPEAN PATENT APPLICATION

(11) **EP 3 560 440 A1**
(43) Date of publication of application: **30.10.2019**
(21) Application number: 19170927.8
(22) Date of filing: 24.04.2019
(51) Int. Cl.: A61B 17/128, A61B 17/00, A61B 17/29

(54) **LATCH ASSEMBLIES AND SURGICAL INSTRUMENTS INCLUDING THE SAME**

(30) Priority: 25.04.2018 US 201862662269 P; 30.01.2019 US 201916262220
(71) Applicant: Covidien LP, Mansfield, MA 02048 (US)
(72) Inventor: BARIL, Jacob C., Norwalk, CT 06851 (US); PILLETERE, Roy J., North Haven, CT 06473 (US); THOMAS, Justin, New Haven, CT 06512 (US); DININO, Matthew A., Newington, CT 064111 (US)
(74) Representative: Maschio, Antonio

(57) **Abstract**

A latch assembly for a surgical instrument, a handle assembly of a surgical instrument including the latch assembly, and a surgical instrument including the latch assembly are provided. The latch assembly includes a lever having a distal engagement section, an intermediate section, and a proximal manipulatable section. The distal engagement section of the lever includes a base and an engagement tooth depending from the base. The engagement tooth is configured to releasably engage first and second components of a surgical instrument with one another and defines a first surface having a concave portion defining a varied radius of curvature.

## Description

### CROSS-REFERENCE TO RELATED APPLICATION

This application claims the benefit of and priority to U.S. Provisional Patent Application No. 62/662,269 filed April 25, 2018, the entire disclosure of which is incorporated by reference herein.

### BACKGROUND

### Technical Field

The present disclosure relates to surgical instruments such as, for example, surgical clip appliers. More particularly, the present disclosure relates to latch assemblies for surgical clip appliers and surgical clip appliers including the same.

### Description of Related Art

Surgical clip appliers are known in the art and are used for a number of distinct and useful surgical procedures. In the case of a laparoscopic surgical procedure, access to the interior of an abdomen is achieved through narrow tubes or cannulas inserted through a small entrance incision in the skin. Minimally invasive procedures performed elsewhere in the body are often generally referred to as endoscopic procedures.

Endoscopic surgical clip appliers having various sizes (e.g., diameters), that are configured to apply a variety of diverse surgical clips, are also known in the art, and are capable of applying a single or multiple surgical clips during an entry to the body cavity. Such surgical clips are typically fabricated from a biocompatible material and are usually compressed over tissue. Once applied to tissue, the compressed surgical clip terminates the flow of fluid therethrough.

### SUMMARY

As detailed herein and shown in the drawing figures, as is traditional when referring to relative positioning on a surgical instrument, the term "proximal" refers to the end of the apparatus or component thereof which is closer to the user and the term "distal" refers to the end of the apparatus or component thereof which is further away from the user. Further, to the extent consistent, any or all of the aspects and features detailed herein may be used in conjunction with any or all of the other aspects and features detailed herein.

Provided in accordance with aspects of the present disclosure is a latch assembly for a surgical instrument including a lever having a distal engagement section, an intermediate section, and a proximal manipulatable section. The distal engagement section of the lever includes a base and an engagement tooth depending from the base. The engagement tooth is configured to releasably engage first and second components of a surgical instrument with one another and defines a first surface having a concave portion defining a varied radius of curvature.

In an aspect of the present disclosure, the latch assembly further includes a pivot pin pivotably supporting the intermediate section of the lever thereon.

In another aspect of the present disclosure, the latch assembly further includes a biasing member operably coupled to the pivot pin.

In yet another aspect of the present disclosure, the radius of curvature of the concave portion varies between a maximum radius of curvature towards ends of the first surface and a minimum radius of curvature towards an intermediate location of the first surface.

In still another aspect of the present disclosure, a ratio of the maximum radius of curvature to the minimum radius of curvature is between 5:1 and 20:1. The ratio of the maximum radius of curvature to the minimum radius of curvature may be between 10:1 and 15:1.

In still yet another aspect of the present disclosure, the engagement tooth defines a generally triangular-shaped configuration including a distal-facing surface extending from the base to an apex. The first surface, in such aspects, is proximally-facing and extends from the base to the apex.

A handle assembly of a surgical instrument provided in accordance with aspects of the present disclosure includes a housing defining a body portion, a fixed handle portion depending from the body portion, and a distal nose extending distally from the body portion, a drive assembly disposed within the housing, and a trigger pivotably connected to the housing and operably associated with the drive assembly. The trigger is movable relative to the fixed handle portion of the housing from an un-actuated position to an actuated position to actuate the drive assembly. The handle assembly further includes a latch assembly operably coupled to the body potion of the housing. The latch assembly is configured to releasably engage an elongated assembly inserted through the distal nose of the housing and may be configured similar to any of the above-detailed aspects or other aspects described herein.

A surgical instrument provided in accordance with aspects of the present disclosure includes an elongated assembly including a proximal hub, a shaft extending distally from the proximal hub, and an end effector extending distally from the shaft. The surgical instrument further includes a handle assembly configured to releasably receive the elongated assembly. The handle assembly includes a housing defining a body portion, a fixed handle portion depending from the body portion, and a distal nose extending distally from the body portion. The handle assembly further includes a drive assembly disposed within the housing and a trigger pivotably connected to the housing and operably associated with the drive assembly. The trigger is movable relative to the fixed handle portion of the housing from an un-actuated position to an actuated position to actuate the drive assembly. The handle assembly further includes a latch assembly operably coupled to the body potion of the housing and configured to releasably engage the elongated assembly upon insertion of the proximal hub of the elongated assembly through the distal nose of the housing. The latch assembly may be configured similar to any of the above-detailed aspects or other aspects described herein.

### BRIEF DESCRIPTION OF THE DRAWINGS

Aspects and features of the presently-disclosed latch assemblies for surgical clip appliers and surgical clip appliers including the same are described in detail with reference to the drawing figures wherein like reference numerals identify similar or identical structural elements and:
FIG. 1 is a first side, perspective view of an endoscopic surgical clip applier provided in accordance with the present disclosure including a handle assembly having an elongated assembly engaged therewith;
FIG. 2 is a second, opposite side, perspective view of a proximal portion of the endoscopic surgical clip applier of FIG. 1 with a portion of the housing removed from the handle assembly to illustrate the internal components therein;
FIG. 3 is a longitudinal, cross-sectional view of the proximal portion of the endoscopic surgical clip applier of FIG. 1;
FIG. 4 is a perspective view of a distal portion of the elongated assembly of the endoscopic surgical clip applier of FIG. 1;
FIG. 5 is a perspective view of a distal portion of another elongated assembly configured for use with the endoscopic surgical clip applier of FIG. 1;
FIG. 6 is a perspective of the endoscopic surgical clip applier of FIG. 1 with the elongated assembly disengaged from the handle assembly;
FIG. 7 is a longitudinal, cross-sectional view of the proximal portion of the endoscopic surgical clip applier of FIG. 1 with the elongated assembly removed;
FIG. 8 is longitudinal, cross-sectional view of a proximal portion of the elongated assembly of FIG. 4, with internal components removed;
FIG. 9 is a top, perspective view of the lever of the latch assembly of the endoscopic surgical clip applier of FIG. 1;
FIG. 10 is an enlarged, perspective view of the distal portion of the lever of FIG. 9; and
FIG. 11 is bottom, perspective view of the lever of FIG. 9.

### DETAILED DESCRIPTION

The present disclosure provides latch assemblies for surgical instruments and surgical instruments including the same. Although detailed herein as incorporated into a surgical clip applier, the latch assemblies of the present disclosure may alternatively be incorporated into any suitable surgical instrument.

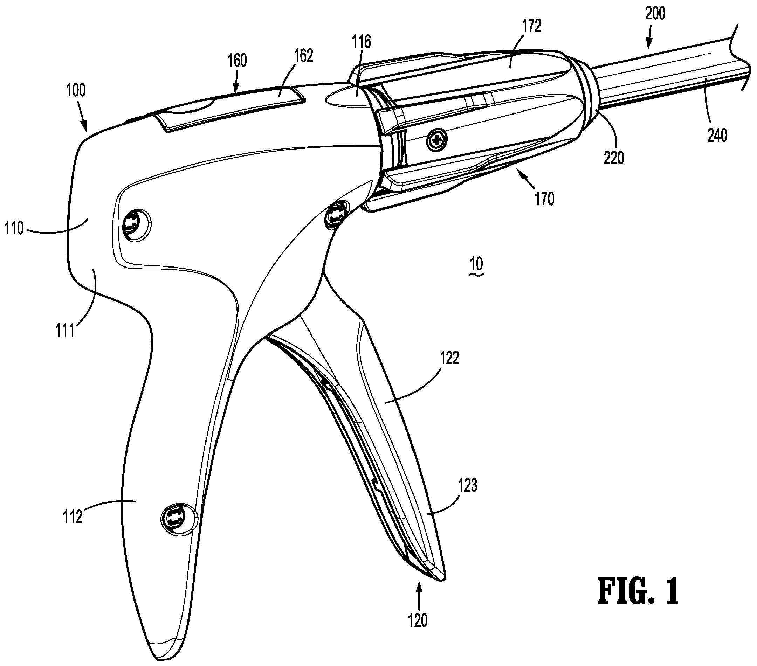

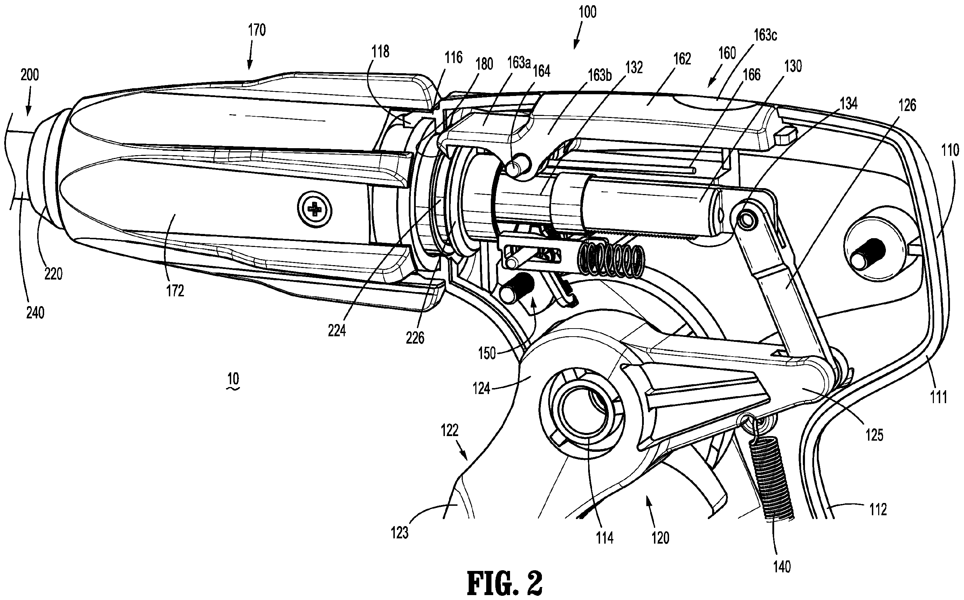

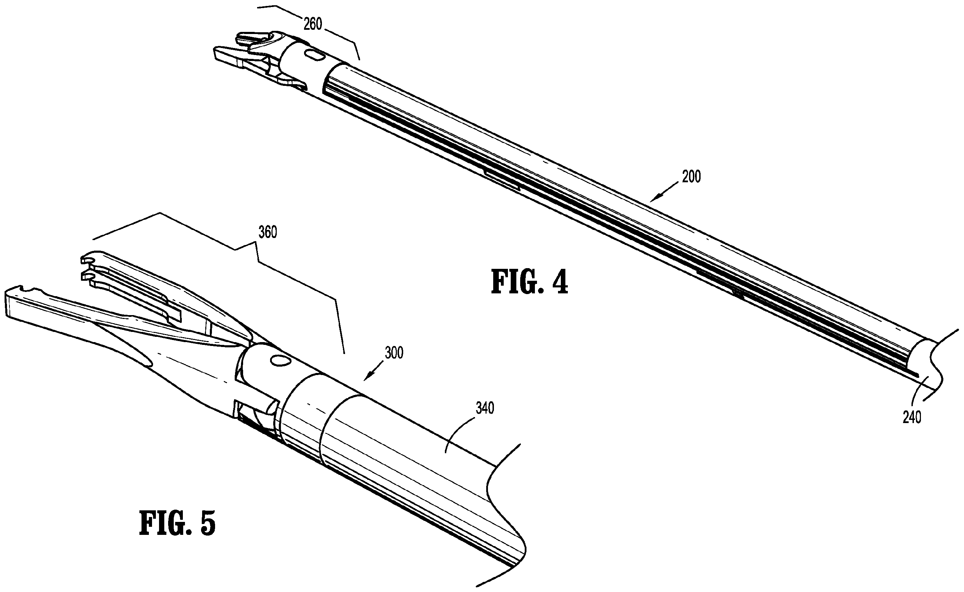

Turning to FIGS. 1-3, an endoscopic surgical clip applier embodying the aspects and features of the present disclosure is shown generally identified by reference numeral 10. Surgical clip applier 10 generally includes a handle assembly 100 and a plurality of elongated assemblies 200 (FIGS. 1-4), 300 (FIG. 5) selectively connectable to handle assembly 100. Handle assembly 100 is configured to operate each of the plurality of elongated assemblies 200, 300 upon connection thereto, and may be configured as a sterilizable, reusable component such that handle assembly 100 may be repeatedly used with different and/or additional elongated assemblies 200, 300 during the course of one or more surgical procedures. The elongated assemblies 200, 300 may be configured as single-use disposable components, limited-use disposable components, or reusable components, depending upon a particular purpose and/or the configuration of the particular elongated assembly. In either configuration, the need for multiple handle assemblies 100 is obviated and, instead, the surgeon need only select an appropriate elongated assembly 200, 300 and connect that elongated assembly to handle assembly 100 in preparation for use.

Handle assembly 100 generally includes a housing 110, an actuation mechanism 120 operably associated with housing 110, a ratchet mechanism 150 operably disposed within housing 110, and a rotation knob assembly 170 operably coupled to a distal portion of housing 110. A latch assembly 160, provided in accordance with the present disclosure, is also operably associated with housing 110, as detailed below. Housing 110 supports and/or encloses the operating components of handle assembly 100. Actuation mechanism 120 is configured to enable selective firing of one or more surgical clips (not shown) from the end effector of the attached elongated assembly. Ratchet mechanical 150 enables ratcheting advancement of drive bar 130 of actuation mechanism 120, when an elongated assembly configured for ratcheting actuation is connected to handle assembly 100. Latch assembly 160 is configured to facilitate releasable locking engagement of the elongated assembly with handle assembly 100. Rotation knob assembly 170 enables the selective rotation of the attached elongated assembly relative to housing 110.

With additional reference to FIGS. 4 and 5, as noted above, handle assembly 100 is configured for use with different elongated assemblies such as, for example, elongated assembly 200 (FIG. 4) and elongated assembly 300 (FIG. 5). Handle assembly 100, more specifically, is configured for both ratcheting use, e.g., in connection with elongated assembly 200 (FIG. 4), and non-ratcheting use, e.g., in connection with elongated assembly 300 (FIG. 5). Elongated assemblies 200, 300 are described briefly below. A more detailed discussion of elongated assemblies, e.g., elongated assemblies 200, 300, configured for use with handle assembly 100 can be found in International Application No. PCT/CN2016/096666, filed on August 26, 2016, International Application No. PCT/CN2016/071178, filed on January 18, 2016, and/or International Application No. PCT/CN2015/091603, filed on October 10, 2015, the entire contents of each of which is hereby incorporated herein by reference.

Referring to FIGS. 4, 6, and 8, in conjunction with FIGS. 1-3, elongated assembly 200 is configured for ratcheting use and generally includes a proximal hub 220, an elongated shaft 240 extending distally from proximal hub 220, an end effector assembly 260 disposed towards a distal end portion of elongated shaft 240, and an inner drive assembly (not shown) operably coupled between handle assembly 100 and end effector assembly 260 when elongated assembly 200 is engaged with handle assembly 100 to enable the sequential firing of at least one surgical clip (not shown) about tissue. End effector assembly 260 of elongated assembly 200 may be configured to fire surgical clips similar to those shown and described in U.S. Patent Nos. 7,819,886 or 7,905,890, the entire contents of each of which is hereby incorporated herein by reference.

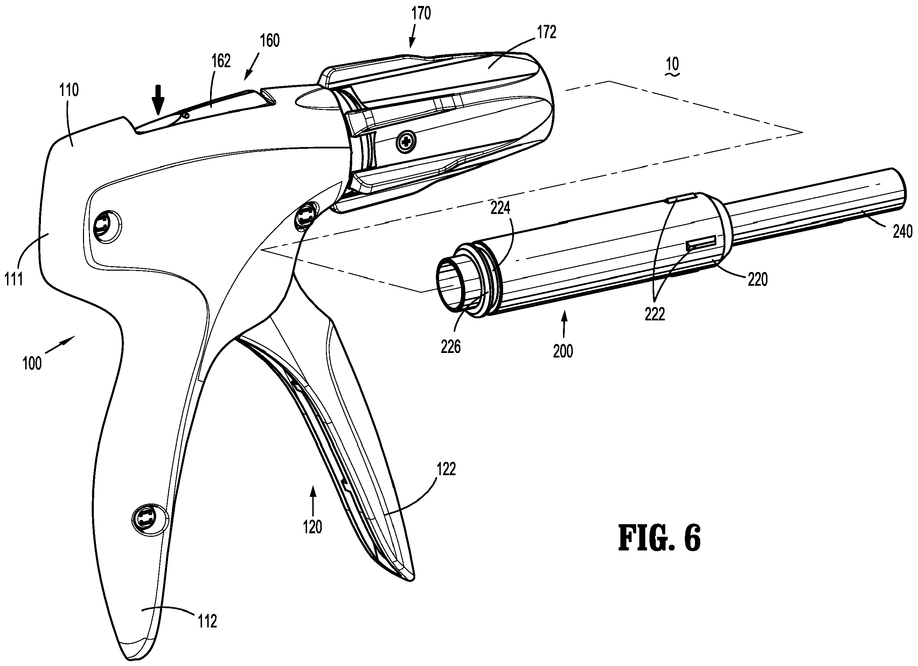

Proximal hub 220 of elongated assembly 200 defines a plurality of indexing protrusions 222 annularly disposed thereabout towards a distal end portion thereof (see FIG. 6). Indexing protrusions 222 are configured for slidable receipt within longitudinally-extending grooves 178 defined within outer knob 172 of rotation knob assembly 170 to rotationally fix proximal hub 220 of elongated assembly 200 relative to rotation knob assembly 170 upon insertion of proximal hub 220 therethrough. As such, in use, rotation of outer knob 172 of rotation knob assembly 170 relative to housing 110 effects corresponding rotation of elongated assembly 200 relative to housing 110.

Proximal hub 220 further defines an annular channel 224 towards the proximal end thereof and a chamfered proximal edge 226. As detailed below, upon insertion of proximal hub 220 through rotation knob assembly 170 and into body portion 111 of housing 110, chamfered proximal edge 226 cams engagement tooth 180 of lever 162 of latch assembly 160 over the outer surface of proximal hub 220 until engagement tooth 180 is disposed in alignment with annular channel 224, whereby engagement tooth 180 falls into engagement within annular recess 224 to engage proximal hub 220 and, thus, elongated assembly 200, with handle assembly 100.

Referring to FIG. 5, elongated assembly 300 is configured for non-ratcheting use and generally includes a proximal hub (not shown), an elongated shaft 340 extending distally from the proximal hub, an end effector assembly 360 disposed towards a distal end portion of elongated shaft 340, and an inner drive assembly (not shown) operably coupled between handle assembly 100 and end effector assembly 360 when elongated assembly 300 is engaged with handle assembly 100 (FIG. 1) to enable grasping and/or manipulation of tissue, retrieval of a surgical clip, and firing of the surgical clip about tissue. It is contemplated that end effector assembly 360 of elongated assembly 300 may be configured to fire surgical clips similar to those shown and described in U.S. Patent No. 4,834,096, the entire contents of which is hereby incorporated herein by reference.

With additional reference to FIGS. 1-3, the proximal hub (not shown) of elongated assembly 300, similarly as with proximal hub 220 of elongated assembly 200, may also include indexing protrusions to rotationally fix elongated assembly 300 relative to rotation knob assembly 170 upon insertion of the proximal hub therethrough and/or an annular channel and chamfered proximal edge to facilitate engagement of engagement tooth 180 of lever 162 of latch assembly 160 with elongated assembly 300 to engage elongated assembly with handle assembly 100.

Although exemplary elongated assemblies 200, 300 configured for ratcheting and non-ratcheting use, respectively, are detailed above, it is contemplated that various other elongated assemblies for performing various different surgical tasks and/or having various different configurations suitable for ratcheting or non-ratcheting use may likewise be utilized with handle assembly 100.

Turning back to FIGS. 1-3, housing 110 of handle assembly 100 may be formed from first and second housing halves that cooperate to define a body portion 111 and a fixed handle portion 112 depending from body portion 111. Body portion 111 of housing 110 includes an internal pivot post 114 extending transversely within body portion 111, and a distal nose 116 defining a distal opening 118 therethrough. A proximal end portion of a proximal hub of an elongated assembly, e.g., proximal hub 220 of elongated assembly 200 or the proximal hub (not shown) of elongated assembly 300 (FIG. 5), is configured to extend at least partially through distal opening 118 of distal nose 116 of housing 110 when the elongated assembly is engaged with handle assembly 100.

Actuation mechanism 120 is operably supported by housing 110 and includes a trigger 122, a linkage 126, a drive bar 130, and a biasing member 140. Trigger 122 includes a grasping portion 123, an intermediate pivot portion 124, and a proximal extension 125. Grasping portion 123 of trigger 122 extends downwardly from body portion 111 of housing 110 in opposed relation relative to fixed handle portion 112 of housing 110. Grasping portion 123 is configured to facilitate grasping and manipulation of trigger 122. Intermediate pivot portion 124 of trigger 122 is at least partially disposed within housing 110 and defines a pivot aperture configured to receive pivot post 114 of housing 110 so as to enable pivoting of trigger 122 about pivot post 114 and relative to housing 110, e.g., between an un-actuated position, wherein grasping portion 123 of trigger 122 is spaced-apart relative to fixed handle portion 112, and an actuated position, wherein grasping portion 123 of trigger 122 is approximated relative to fixed handle portion 112.

Proximal extension 125 of trigger 122 is disposed on an opposite side of intermediate pivot portion 124 and, thus, pivot post 114, as compared to grasping portion 123 of trigger 122. As such, pivoting of grasping portion 123 to rotate in one direction, e.g., proximally towards fixed handle portion 112, pivots proximal extension 125 to rotate in the opposite direction, e.g., distally. Proximal extension 125 of trigger 122 is pivotably coupled to the proximal end of linkage 126. Biasing member 140 is secured at either end and extends between proximal extension portion 125 of trigger 122 and a support (not shown) disposed within fixed handle portion 112 of housing 110. Pivoting of grasping portion 123 towards the actuated position elongates biasing member 140 storing energy therein such that, upon release of grasping portion 123, grasping portion 123 is returned towards the un-actuated position under the bias of biasing member 140. Although illustrated as an extension coil spring, biasing member 140 may define any suitable configuration for biasing grasping portion 123 of trigger 122 towards the un-actuated position.

As noted above, linkage 126 is coupled at its proximal end to proximal extension portion 125 of trigger 122. Linkage 126 is also pivotably coupled at its distal end to a proximal end of drive bar 130. As a result of this configuration, pivoting of grasping portion 123 of trigger 122 towards the actuated position urges proximal extension portion 125 of trigger 122 distally which, in turn, urges linkage 126 distally to, in turn, urge drive bar 130 distally.

Drive bar 130 is slidable through body portion 111 of housing 110, in response to actuation of trigger 122, to urge a distal end portion 132 of drive bar 130 into contact with a proximal actuator of an inner drive assembly (not shown) of an elongated assembly, e.g., elongated assembly 200 or elongated assembly 300 (FIG. 5), engaged with handle assembly 100 to fire a surgical clip supported at the end effector assembly of the elongated assembly. Drive bar 130, more specifically, is slidable from an un-actuated, proximal position, corresponding to the un-actuated position of grasping portion 123 of trigger 122, to an actuated, distal position, corresponding to the actuated position of grasping portion 123 of trigger 122, in order to urge the proximal actuator of the inner drive assembly (not shown) of the elongated assembly distally to fire a surgical clip supported at the end effector assembly of the elongated assembly.

Drive bar 130 may further include a ratchet rack 134 extending along at least a portion of an underside surface thereof. Ratchet rack 134 is configured to selectively interface with ratchet mechanism 150 to enable advancement of drive bar 130 in either a ratcheting condition or a non-ratcheting condition. Ratchet rack 134 and ratchet mechanism 150 may be configured similarly as described in, for example, International Application No. PCT/CN2016/096666 or International Application No. PCT/CN2016/071178, each of which was previously incorporated by reference herein.

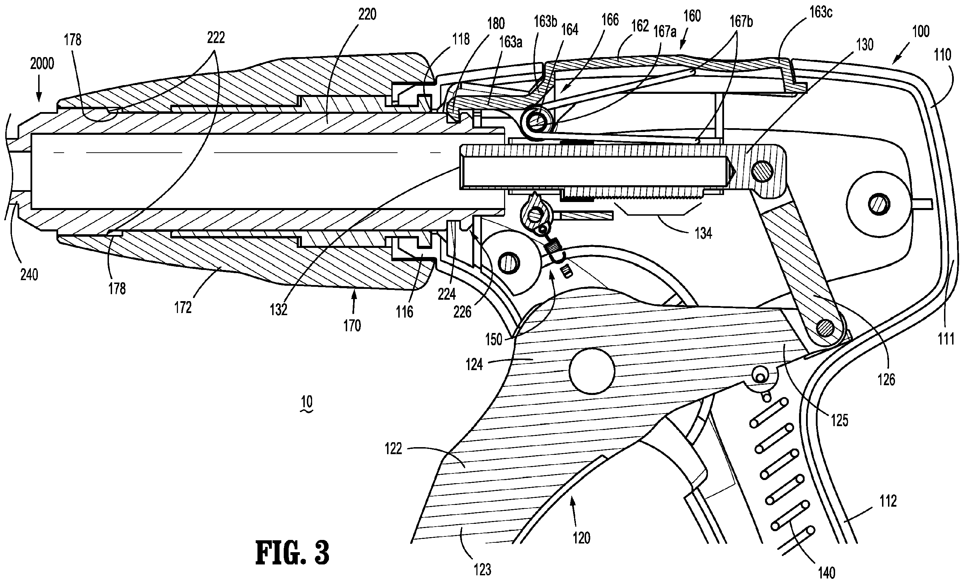

Continuing with reference to FIGS. 1-3, rotation knob assembly 170 is rotatably coupled to distal nose 116 of body portion 111 of housing 110 and is configured to receive the proximal hub of the elongated assembly, e.g., proximal hub 220 of elongated assembly 200, coupled to handle assembly 100 in fixed rotational engagement therewith, e.g., via receipt of indexing protrusions 222 within grooves 178 (see FIG. 3), to enable selective rotation of elongated assembly 200 relative to housing 110 upon rotation of outer knob 172 of rotation knob assembly 170 relative to housing 110.

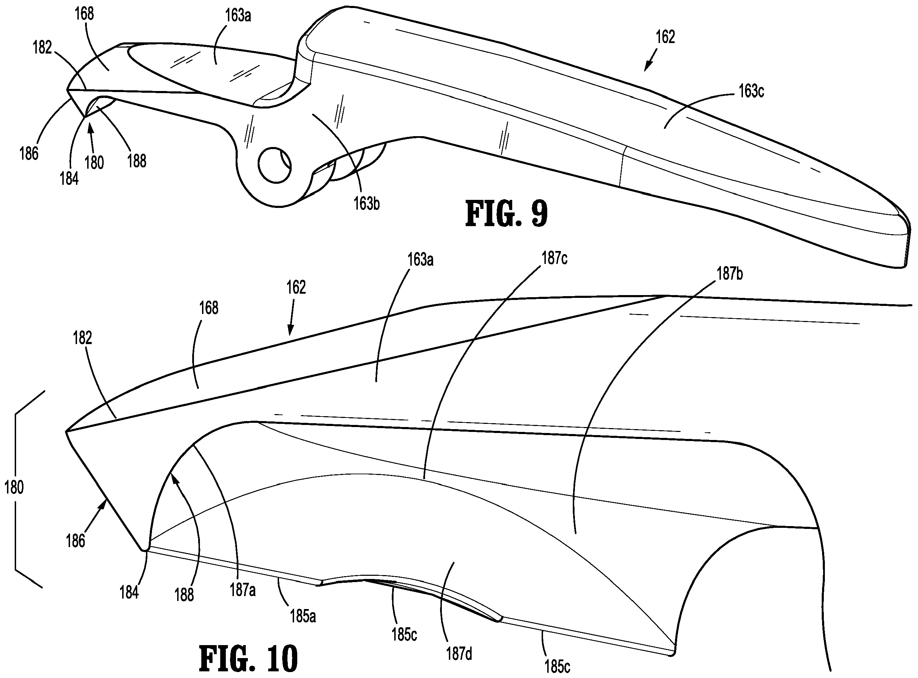

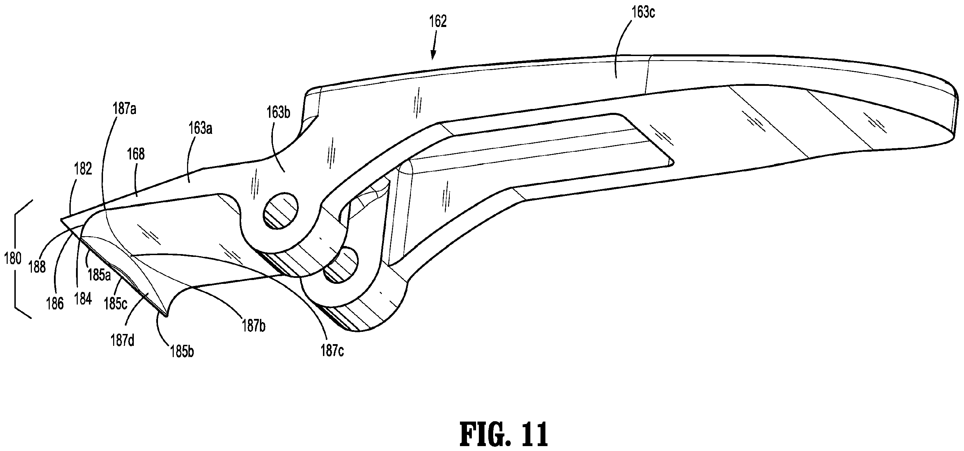

Referring now to FIGS. 1-3, 6, and 7, latch assembly 160 of the present disclosure includes a lever 162, a pivot pin 164, and a biasing member 166. Lever 162 is at least partially disposed within a cut-out defined without housing 110 of handle assembly 100 to enable manual manipulation thereof and defines a distal engagement section 163a, an intermediate section 163b, and a proximal manipulatable section 163c. Distal engagement section 163a of lever 162 includes an engagement tooth 180 extending therefrom. Engagement tooth 180 is configured to engage an elongated assembly, e.g., elongated assembly 200, inserted into handle assembly 100. More specifically, as noted above with respect to elongated assembly 200, for example, upon insertion of proximal hub 220 of elongated assembly 200 into handle assembly 100, engagement tooth 180 is configured to cam over chamfered proximal edge 226 of proximal hub 220 and into engagement within annular channel 224 to thereby lock elongated assembly 200 in engagement with handle assembly 100. Lever 162 and, in particular, distal engagement section 163a thereof, is described in greater detail below with reference to FIGS. 9-11.

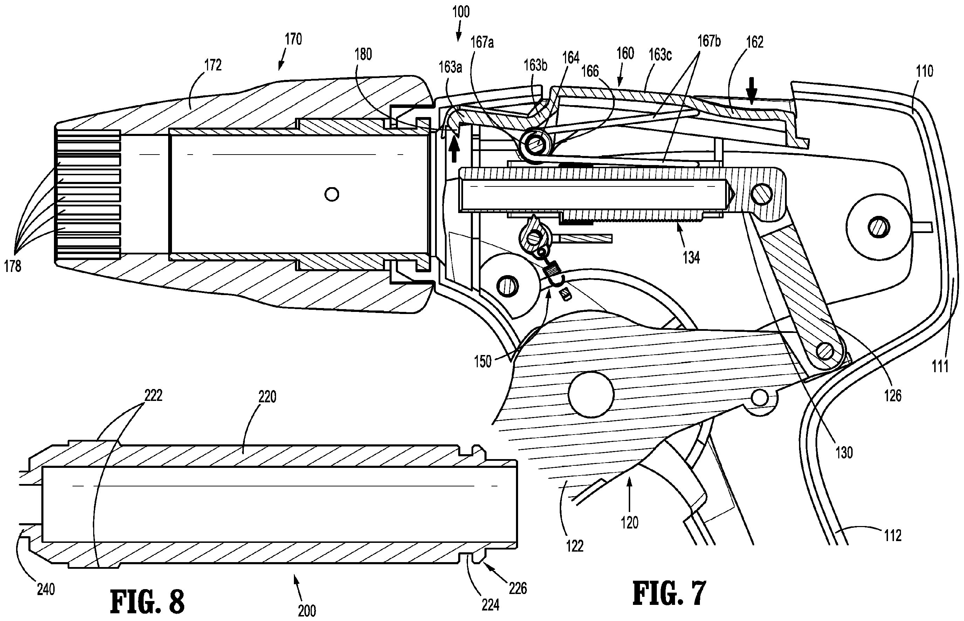

Pivot pin 164 of latch assembly 160 pivotably couples intermediate section 163b of lever 162 with housing 110 of handle assembly 100 such that urging of proximal manipulation section 163c of lever 162 in a first direction into housing 110, urges distal engagement section 163a of lever 162 in a second, opposite direction out of engagement with annular channel 224 of proximal hub 220 of elongated assembly 200. Biasing member 166 is configured as a torsion spring having a body 167a disposed about pivot pin 164 and first and second legs 167b disposed between housing 110 and proximal manipulation section 163c of lever 162 to bias proximal manipulation section 163c of lever 162 away from housing 110, thereby biasing distal engagement section 163a towards an engaged position. However, other suitable configurations of biasing member 166 are also contemplated. Proximal manipulation section 163c of lever 162 is selectively depressible into housing 110, as illustrated in FIGS. 6 and 7, against the bias of biasing member 166 to urge distal engagement section 163a towards a disengaged position to disengage engagement tooth 180 from annular channel 224 of proximal hub 220 of elongated assembly 200 and enable withdrawal of elongated assembly 200 from handle assembly 100.

Turning now to FIGS. 9-11, the engagement of engagement tooth 180 of distal engagement section 163a of lever 162 of latch assembly 160 within the annular channel of a proximal hub of an elongated assembly, e.g., annular channel 224 of proximal hub 222 of elongated assembly 200 (FIGS. 1-3 and 8) or the annular channel of any other elongated assembly configured for use with handle assembly 100 (FIGS. 1-3), retains the elongated assembly in operable engagement with handle assembly 100 (FIGS. 1-3), which is critical for proper operation. As such, it is a consideration to ensure that lever 162 provides proper retention in all use conditions, with all elongated assemblies, and throughout the entire life of handle assembly 100 FIGS. 1-3), during which handle assembly 100 may go through a large number of engagement, firing, and disengagement use cycles and a large number of cleaning/autoclaving cycles. It is also a consideration that the space occupied by lever 162 within handle assembly 100 is minimized to maintain the ergonomics of handle assembly 100 and such that lever 162 does not interfere with the other operable components within handle assembly 100. Distal engagement section 163a of lever 162, detailed below, meets both of these considerations.

Distal engagement section 163a of lever 162 includes a base portion 168 having engagement tooth 180 depending therefrom in generally perpendicular orientation relative thereto. Engagement tooth 180, more specifically, defines a generally triangular cross-sectional configuration wherein engagement tooth 180 defines a maximum width at a base 182 (positioned adjacent to base portion 168 of distal engagement section 163a) and tapers from the maximum width at base 182 to a minimal width at an apex 184. Engagement tooth 180 further defines a distally-facing, outer surface 186 extending between base 182 and apex 184 on a distal side of engagement tooth 180 and a proximally-facing, inner surface 188 extending between base 182 and apex 184 on a proximal side of engagement tooth 180. Apex 184 of engagement tooth 180 may define a pair of linear segments 185a, 185b having an arc-shaped cut-out 185c defined therebetween. Arc-shaped cut-out 185c may be centrally disposed between the opposed ends of apex 184 of engagement tooth 180 such that linear segments 185a, 185b define equal lengths, although other configurations are also contemplated.

Distally-facing, outer surface 186 of engagement tooth 180 is configured to cam over chamfered proximal edge 226 of proximal hub 220 of elongated assembly 200 upon insertion of elongated assembly 200 into handle assembly 100 to facilitate engagement of elongated assembly 200 within handle assembly 100, as noted above (see FIGS. 3, 7, and 8). To this end, distally-facing, outer surface 186 may define a generally planar configuration (within manufacturing and material tolerances) sloped in a distal-to-proximal direction from base 182 of engagement tooth 180 to apex 184 thereof. In embodiments, the slope of outer surface 186 is equal in magnitude to the slope of chamfered proximal edge 226 of proximal hub 220 of elongated assembly 200 to facilitate camming of engagement tooth 180 about proximal hub 220 and into engagement within annular channel 224 (see FIGS. 3, 7, and 8).

Proximally-facing, inner surface 188 of engagement tooth 180 defines a concave configuration over a portion thereof; the concave portion having a varied radius of curvature. More specifically, the concave portion of proximally-facing inner surface 188 defines a first, maximum radius of curvature at first and second ends 187a, 187b, respectively, thereof, wherein the concave portion extends the entire width of proximally-facing, inner surface 188 at the ends 187a, 187b. The concave portion of proximally-facing, inner surface 188 further defines a second, minimum radius of curvature at an intermediate location 187c such that the concave portion extends only a portion of the width of proximally-facing, inner surface 188 at intermediate location 187c. Intermediate location 187c may be centrally located between first and second ends 187a, 187b, respectively, or otherwise disposed therebetween.

The radius of curvature of the concave portion of proximally-facing inner surface 188 may decrease continuously and smoothly from the ends 187a, 187b to the intermediate location 187c. The radius of curvature at the ends 187a, 187b and the radius of curvature at the intermediate location 187c may define a ratio, in embodiments, of 5:1 to 20:1, in other embodiments, of 10:1 to 15:1, and, in other embodiments, of 12:1 to 13:1. In embodiments, the radii of curvature may define a ratio of 12.5:1, and, in such embodiments, the radius of curvature at the end 187a, 187b may be about 0.0625 inches (within manufacturing an material tolerances) and the radius of curvature at the intermediate location 187c may be about 0.005 inches (within manufacturing and material tolerances).

Proximally-facing, inner surface 188 further defines a planar portion 187d that occupies the remainder of proximally-facing, inner surface 188 (e.g., the portion that is not part of the concave portion), although this remainder portion may also be curved or otherwise configured. Planar portion 187d defines an inverse configuration relative to the concave portion. That is, planar portion 187d defines a minimum or zero width adjacent ends 187a, 187b and a maximum width adjacent intermediate portion 187c.

The above-detailed configuration of engagement tooth 180 and, more specifically, proximally-facing, inner surface 188 thereof provides a secure engagement between lever 162 and a proximal hub of an elongated assembly, is capable of withstanding multiple use and cleaning/sterilization cycles, and enables a reduction in the overall thickness of engagement tooth 180 without compromising mechanical stability. More specifically, the thickness of engagement tooth 180 has been shown to provide the same mechanical stability as an engagement tooth without the above-described features of proximally-facing, inner surface 188 that has a 10% greater thickness.

With general reference back to FIGS 1-4, 6, and 7, insertion and engagement of an elongated assembly, e.g., elongated assembly 200, with handle assembly 100 and use of the same are described. In order to engage elongated assembly 200 with handle assembly 100, proximal hub 220 of elongated assembly 200 is inserted through outer knob 172 of rotation knob assembly 170 and into distal nose 116 of housing 110, wherein engagement tooth 180 of latch assembly 160 cams over chamfered proximal edge 226 of proximal hub 220 and into engagement within annular channel 224 of proximal hub 220 to thereby rotatably engage proximal hub 220 relative to housing 110. Upon insertion of proximal hub 220 through rotation knob assembly 170, indexing protrusions 222 of proximal hub 220 are received within longitudinally-extending grooves 178 of outer knob 172 to rotationally fix proximal hub 220 relative to outer knob 172.

With elongated assembly 200 engaged with handle assembly 100 as detailed above, handle assembly 100 may be manipulated and/or outer knob 172 rotated to position end effector 260 (FIG. 4) of elongated assembly 200 about tissue to be treated. Once end effector 260 is positioned as desired, trigger 122 is pivoted towards fixed handle portion 112 of housing 110 to urge linkage 126 distally which, in turn, urges drive bar 130 distally through housing 110 to drive the inner drive assembly (not shown) of elongated assembly 200 distally through elongated assembly 200 to fire and form a surgical clip from end effector assembly 260 (FIG. 3A) about tissue. The above may be repeated to fire and form several surgical clips about tissue, as necessary.

In order to disengage elongated assembly 200 from handle assembly 100, e.g., for cleaning and/or sterilization, or to replace elongated assembly 200 with another elongated assembly, lever 162 of latch assembly 160 is depressed inwardly into housing 110 to disengage engagement tooth 180 from annular channel 224, thereby disengaging lever 162 from proximal hub 220 of elongated assembly 200 and enabling proximal hub 220 to be withdrawn distally from housing 110 and rotation knob assembly 170.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods and techniques that are insubstantially different from those described above and/or in the appended claims are also intended to be within the scope of the disclosure.

The invention may be described by reference to the following numbered paragraphs:-
1. A latch assembly for a surgical instrument, comprising:
   a lever including a distal engagement section, an intermediate section, and a proximal manipulatable section, the distal engagement section including a base and an engagement tooth depending from the base, the engagement tooth configured to releasably engage first and second components of a surgical instrument with one another, the engagement tooth defining a first surface having a concave portion defining a varied radius of curvature.
2. The latch assembly according to paragraph 1, wherein the radius of curvature of the concave portion varies between a maximum radius of curvature towards ends of the first surface and a minimum radius of curvature towards an intermediate location of the first surface.
3. The latch assembly according to paragraph 2, wherein a ratio of the maximum radius of curvature to the minimum radius of curvature is between 5:1 and 20:1.
4. The latch assembly according to paragraph 2, wherein a ratio of the maximum radius of curvature to the minimum radius of curvature is between 10:1 and 15:1.
5. The latch assembly according to paragraph 2, further comprising a pivot pin pivotably supporting the intermediate section of the lever thereon.
6. The latch assembly according to paragraph 5, further comprising a biasing member operably coupled to the pivot pin.
7. The latch assembly according to paragraph 1, wherein the engagement tooth defines a generally triangular-shaped configuration including a distal-facing surface extending from the base to an apex, and wherein the first surface is proximally-facing extending from the base to the apex
8. A handle assembly of a surgical instrument, comprising:
   a housing defining a body portion, a fixed handle portion depending from the body portion, and a distal nose extending distally from the body portion;
   a drive assembly disposed within the housing;
   a trigger pivotably connected to the housing and operably associated with the drive assembly, the trigger movable relative to the fixed handle portion of the housing from an un-actuated position to an actuated position to actuate the drive assembly; and
   a latch assembly operably coupled to the body potion of the housing, the latch assembly configured to releasably engage an elongated assembly inserted through the distal nose of the housing, the latch assembly including:
      a lever including a distal engagement section, an intermediate section, and a proximal manipulatable section, the distal engagement section including a base and an engagement tooth depending from the base, the engagement tooth configured to releasably engage the elongated assembly, the engagement tooth defining a first surface having a concave portion defining a varied radius of curvature.
9. The handle assembly according to paragraph 8, wherein the radius of curvature of the concave portion varies between a maximum radius of curvature towards ends of the first surface and a minimum radius of curvature towards an intermediate location of the first surface.
10. The handle assembly according to paragraph 9, wherein a ratio of the maximum radius of curvature to the minimum radius of curvature is between 5:1 and 20:1.
11. The handle assembly according to paragraph 9, wherein a ratio of the maximum radius of curvature to the minimum radius of curvature is between 10:1 and 15:1.
12. The handle assembly according to paragraph 9, wherein the latch assembly further includes a pivot pin pivotably coupling the intermediate section of the lever with the housing.
13. The handle assembly according to paragraph 12, wherein the latch assembly further includes a biasing member operably coupled to the pivot pin and configured to bias the latch towards an engaged position.
14. The handle assembly according to paragraph 8, wherein the engagement tooth defines a generally triangular-shaped configuration including a distal-facing surface extending from the base to an apex, and wherein the first surface is proximally-facing extending from the base to the apex
15. A surgical instrument, comprising:
   an elongated assembly including a proximal hub, a shaft extending distally from the proximal hub, and an end effector extending distally from the shaft; and
   a handle assembly, including:
      a housing defining a body portion, a fixed handle portion depending from the body portion, and a distal nose extending distally from the body portion;
      a drive assembly disposed within the housing;
      a trigger pivotably connected to the housing and operably associated with the drive assembly, the trigger movable relative to the fixed handle portion of the housing from an un-actuated position to an actuated position to actuate the drive assembly; and
      a latch assembly operably coupled to the body potion of the housing, the latch assembly configured to releasably engage the elongated assembly upon insertion of the proximal hub of the elongated assembly through the distal nose of the housing, the latch assembly including:
         a lever including a distal engagement section, an intermediate section, and a proximal manipulatable section, the distal engagement section including a base and an engagement tooth depending from the base, the engagement tooth configured to releasably engage a channel defined within the proximal hub of the elongated assembly, the engagement tooth defining a first surface having a concave portion defining a varied radius of curvature.
16. The surgical instrument according to paragraph 15, wherein the radius of curvature of the concave portion varies between a maximum radius of curvature towards ends of the first surface and a minimum radius of curvature towards an intermediate location of the first surface.
17. The surgical instrument according to paragraph 16, wherein a ratio of the maximum radius of curvature to the minimum radius of curvature is between 5:1 and 20:1.
18. The surgical instrument according to paragraph 16, wherein a ratio of the maximum radius of curvature to the minimum radius of curvature is between 10:1 and 15:1.
19. The surgical instrument according to paragraph 15, wherein the engagement tooth defines a generally triangular-shaped configuration including a distal-facing surface extending from the base to an apex, and wherein the first surface is proximally-facing extending from the base to the apex
20. The surgical instrument according to paragraph 15, wherein the latch assembly further comprises:
   a pivot pin pivotably coupling the intermediate section of the lever with the housing; and
   a biasing member operably coupled to the pivot pin and configured to bias the latch towards an engaged position.

## Claims

1. A latch assembly for a surgical instrument, comprising:
a lever including a distal engagement section, an intermediate section, and a proximal manipulatable section, the distal engagement section including a base and an engagement tooth depending from the base, the engagement tooth configured to releasably engage first and second components of a surgical instrument with one another, the engagement tooth defining a first surface having a concave portion defining a varied radius of curvature.

2. The latch assembly according to claim 1, wherein the radius of curvature of the concave portion varies between a maximum radius of curvature towards ends of the first surface and a minimum radius of curvature towards an intermediate location of the first surface.

3. The latch assembly according to claim 2, wherein a ratio of the maximum radius of curvature to the minimum radius of curvature is between 5:1 and 20:1; preferably wherein a ratio of the maximum radius of curvature to the minimum radius of curvature is between 10:1 and 15:1.

4. The latch assembly according to claim 2, further comprising a pivot pin pivotably supporting the intermediate section of the lever thereon; and/or further comprising a biasing member operably coupled to the pivot pin.

5. The latch assembly according to any preceding claim, wherein the engagement tooth defines a generally triangular-shaped configuration including a distal-facing surface extending from the base to an apex, and wherein the first surface is proximally-facing extending from the base to the apex

6. A handle assembly of a surgical instrument, comprising:
a housing defining a body portion, a fixed handle portion depending from the body portion, and a distal nose extending distally from the body portion;
a drive assembly disposed within the housing;
a trigger pivotably connected to the housing and operably associated with the drive assembly, the trigger movable relative to the fixed handle portion of the housing from an un-actuated position to an actuated position to actuate the drive assembly; and
a latch assembly operably coupled to the body potion of the housing, the latch assembly configured to releasably engage an elongated assembly inserted through the distal nose of the housing, the latch assembly including:
a lever including a distal engagement section, an intermediate section, and a proximal manipulatable section, the distal engagement section including a base and an engagement tooth depending from the base, the engagement tooth configured to releasably engage the elongated assembly, the engagement tooth defining a first surface having a concave portion defining a varied radius of curvature.

7. The handle assembly according to claim 6, wherein the radius of curvature of the concave portion varies between a maximum radius of curvature towards ends of the first surface and a minimum radius of curvature towards an intermediate location of the first surface.

8. The handle assembly according to claim 6 or claim 7, wherein a ratio of the maximum radius of curvature to the minimum radius of curvature is between 5:1 and 20:1; preferably wherein a ratio of the maximum radius of curvature to the minimum radius of curvature is between 10:1 and 15:1.

9. The handle assembly according to any of claims 6 to 8, wherein the latch assembly further includes a pivot pin pivotably coupling the intermediate section of the lever with the housing; preferably wherein the latch assembly further includes a biasing member operably coupled to the pivot pin and configured to bias the latch towards an engaged position.

10. The handle assembly according to any of claims 6 to 9, wherein the engagement tooth defines a generally triangular-shaped configuration including a distal-facing surface extending from the base to an apex, and wherein the first surface is proximally-facing extending from the base to the apex.

11. A surgical instrument, comprising:
an elongated assembly including a proximal hub, a shaft extending distally from the proximal hub, and an end effector extending distally from the shaft; and
a handle assembly, including:
a housing defining a body portion, a fixed handle portion depending from the body portion, and a distal nose extending distally from the body portion;
a drive assembly disposed within the housing;
a trigger pivotably connected to the housing and operably associated with the drive assembly, the trigger movable relative to the fixed handle portion of the housing from an un-actuated position to an actuated position to actuate the drive assembly; and
a latch assembly operably coupled to the body potion of the housing, the latch assembly configured to releasably engage the elongated assembly upon insertion of the proximal hub of the elongated assembly through the distal nose of the housing, the latch assembly including:
a lever including a distal engagement section, an intermediate section, and a proximal manipulatable section, the distal engagement section including a base and an engagement tooth depending from the base, the engagement tooth configured to releasably engage a channel defined within the proximal hub of the elongated assembly, the engagement tooth defining a first surface having a concave portion defining a varied radius of curvature.

12. The surgical instrument according to claim 11, wherein the radius of curvature of the concave portion varies between a maximum radius of curvature towards ends of the first surface and a minimum radius of curvature towards an intermediate location of the first surface.

13. The surgical instrument according to claim 11 or claim 12, wherein a ratio of the maximum radius of curvature to the minimum radius of curvature is between 5:1 and 20:1; preferably wherein a ratio of the maximum radius of curvature to the minimum radius of curvature is between 10:1 and 15:1.

14. The surgical instrument according to any of claims 11 to 13, wherein the engagement tooth defines a generally triangular-shaped configuration including a distal-facing surface extending from the base to an apex, and wherein the first surface is proximally-facing extending from the base to the apex

15. The surgical instrument according to any of claims 11 to 14, wherein the latch assembly further comprises:
a pivot pin pivotably coupling the intermediate section of the lever with the housing; and
a biasing member operably coupled to the pivot pin and configured to bias the latch towards an engaged position.
